# EUROPEAN PATENT APPLICATION

(11) **EP 3 002 589 A1**
(43) Date of publication of application: **06.04.2016**
(21) Application number: 15187839.4
(22) Date of filing: 01.10.2015
(51) Int. Cl.: G01N 33/574, G01N 33/74

(54) **A METHOD FOR STRATIFYING A FEMALE SUBJECT FOR HORMONE REPLACEMENT THERAPY**

(30) Priority: 01.10.2014 EP 14187316
(71) Applicant: sphingotec GmbH, 16761 Henningsdorf (DE)
(72) Inventor: BERGMANN, Andreas, 13465 Berlin (DE); MELANDER, Olle, 21611 Limhamn (SE)
(74) Representative: Kilger, Ute

(57) **Abstract**

Subject matter of the present invention is a method for stratifying a female subject for hormone replacement therapy comprising:
• determining the level of Pro-Neurotensin or fragments thereof of at least 5 amino acids in a bodily fluid obtained from said female subject; and
• comparing said level of Pro-Neurotensin or fragments thereof with a pre-determined pro-NT threshold and
• wherein in case the determined level of Pro-Neurotensin or fragments thereof is above said pre-determined threshold then the subject is identified as having an enhanced risk to suffer an adverse event or having an enhanced risk to attract a disease in case of a hormone replacement therapy, and
• wherein in case the determined level of Pro-Neurotensin or fragments thereof is below said pre-determined threshold then the subject is identified as not having an enhanced risk to suffer an adverse event or as not having an enhanced risk to attract a disease in case of a hormone replacement therapy, and/or
• determining the level of Pro-Enkephalin or fragments thereof of at least 5 amino acids (pENK) in a bodily fluid obtained from said female subject; and
• comparing said level of Pro-Enkephalin or fragments thereof with a pre-determined "pENK threshold" and
• wherein in case the determined level of Pro-Enkephalin or fragments thereof is below said pre-determined pENK threshold then the subject is identified as having an enhanced risk to suffer an adverse event or having an enhanced risk to attract a disease in case of a hormone replacement therapy, and
• wherein in case the determined level of Pro-Enkephalin or fragments thereof is above said pre-detennined pENK threshold then the subject is identified as not having an enhanced risk to suffer an adverse event or as not having an enhanced risk to attract a disease in case of a hormone replacement therapy.

SEQUENCE LISTING

<110>  sphingotec GmbH

<120>  A method for stratifying a female subject for hormone replacement
       therapy

<130>  S75038EP01

<160>  29

<170>  PatentIn version 3.3

<210>  1
<211>  147
<212>  PRT
<213>  Homo sapiens

<400>  1

Ser Asp Ser Glu Glu Glu Met Lys Ala Leu Glu Ala Asp Phe Leu Thr
1               5                   10                  15

Asn Met His Thr Ser Lys Ile Ser Lys Ala His Val Pro Ser Trp Lys
            20                  25                  30

Met Thr Leu Leu Asn Val Cys Ser Leu Val Asn Asn Leu Asn Ser Pro
        35                  40                  45

Ala Glu Glu Thr Gly Glu Val His Glu Glu Glu Leu Val Ala Arg Arg
    50                  55                  60

Lys Leu Pro Thr Ala Leu Asp Gly Phe Ser Leu Glu Ala Met Leu Thr
65                  70                  75                  80

Ile Tyr Gln Leu His Lys Ile Cys His Ser Arg Ala Phe Gln His Trp
                85                  90                  95

Glu Leu Ile Gln Glu Asp Ile Leu Asp Thr Gly Asn Asp Lys Asn Gly
            100                 105                 110

Lys Glu Glu Val Ile Lys Arg Lys Ile Pro Tyr Ile Leu Lys Arg Gln
        115                 120                 125

Leu Tyr Glu Asn Lys Pro Arg Arg Pro Tyr Ile Leu Lys Arg Asp Ser
    130                 135                 140

Tyr Tyr Tyr
145

<210>  2
<211>  125
<212>  PRT

<213>  Homo sapiens

<400>  2

Ser Asp Ser Glu Glu Glu Met Lys Ala Leu Glu Ala Asp Phe Leu Thr
1               5                   10                  15

Asn Met His Thr Ser Lys Ile Ser Lys Ala His Val Pro Ser Trp Lys
            20                  25                  30

Met Thr Leu Leu Asn Val Cys Ser Leu Val Asn Asn Leu Asn Ser Pro
        35                  40                  45

Ala Glu Glu Thr Gly Glu Val His Glu Glu Glu Leu Val Ala Arg Arg
    50                  55                  60

Lys Leu Pro Thr Ala Leu Asp Gly Phe Ser Leu Glu Ala Met Leu Thr
65                  70                  75                  80

Ile Tyr Gln Leu His Lys Ile Cys His Ser Arg Ala Phe Gln His Trp
                85                  90                  95

Glu Leu Ile Gln Glu Asp Ile Leu Asp Thr Gly Asn Asp Lys Asn Gly
            100                 105                 110

Lys Glu Glu Val Ile Lys Arg Lys Ile Pro Tyr Ile Leu
        115                 120                 125

<210>  3
<211>  6
<212>  PRT
<213>  Homo sapiens

<400>  3

Lys Ile Pro Tyr Ile Leu
1               5

<210>  4
<211>  13
<212>  PRT
<213>  Homo sapiens

<400>  4

Gln Leu Tyr Glu Asn Lys Pro Arg Arg Pro Tyr Ile Leu
1               5                   10

<210>  5
<211>  117
<212>  PRT
<213>  Homo sapiens

<400>  5

Ser Asp Ser Glu Glu Glu Met Lys Ala Leu Glu Ala Asp Phe Leu Thr
1               5                   10                  15

Asn Met His Thr Ser Lys Ile Ser Lys Ala His Val Pro Ser Trp Lys
            20                  25                  30

Met Thr Leu Leu Asn Val Cys Ser Leu Val Asn Asn Leu Asn Ser Pro
        35                  40                  45

Ala Glu Glu Thr Gly Glu Val His Glu Glu Glu Leu Val Ala Arg Arg
    50                  55                  60

Lys Leu Pro Thr Ala Leu Asp Gly Phe Ser Leu Glu Ala Met Leu Thr
65                  70                  75                  80

Ile Tyr Gln Leu His Lys Ile Cys His Ser Arg Ala Phe Gln His Trp
                85                  90                  95

Glu Leu Ile Gln Glu Asp Ile Leu Asp Thr Gly Asn Asp Lys Asn Gly
            100                 105                 110

Lys Glu Glu Val Ile
        115

<210>  6
<211>  132
<212>  PRT
<213>  Homo sapiens

<400>  6

Ser Asp Ser Glu Glu Glu Met Lys Ala Leu Glu Ala Asp Phe Leu Thr
1               5                   10                  15

Asn Met His Thr Ser Lys Ile Ser Lys Ala His Val Pro Ser Trp Lys
            20                  25                  30

Met Thr Leu Leu Asn Val Cys Ser Leu Val Asn Asn Leu Asn Ser Pro
        35                  40                  45

Ala Glu Glu Thr Gly Glu Val His Glu Glu Glu Leu Val Ala Arg Arg
    50                  55                  60

Lys Leu Pro Thr Ala Leu Asp Gly Phe Ser Leu Glu Ala Met Leu Thr
65                  70                  75                  80

Ile Tyr Gln Leu His Lys Ile Cys His Ser Arg Ala Phe Gln His Trp
                85                  90                  95

Glu Leu Ile Gln Glu Asp Ile Leu Asp Thr Gly Asn Asp Lys Asn Gly
            100                 105                 110

Lys Glu Glu Val Ile Lys Arg Lys Ile Pro Tyr Ile Leu Lys Arg Gln
        115                 120                 125

Leu Tyr Glu Asn
    130

<210>  7
<211>  125
<212>  PRT
<213>  Homo sapiens

<400>  7

Ser Asp Ser Glu Glu Glu Met Lys Ala Leu Glu Ala Asp Phe Leu Thr
1               5                   10                  15

Asn Met His Thr Ser Lys Ile Ser Lys Ala His Val Pro Ser Trp Lys
            20                  25                  30

Met Thr Leu Leu Asn Val Cys Ser Leu Val Asn Asn Leu Asn Ser Pro
        35                  40                  45

Ala Glu Glu Thr Gly Glu Val His Glu Glu Glu Leu Val Ala Arg Arg
    50                  55                  60

Lys Leu Pro Thr Ala Leu Asp Gly Phe Ser Leu Glu Ala Met Leu Thr
65                  70                  75                  80

Ile Tyr Gln Leu His Lys Ile Cys His Ser Arg Ala Phe Gln His Trp
                85                  90                  95

Glu Leu Ile Gln Glu Asp Ile Leu Asp Thr Gly Asn Asp Lys Asn Gly
            100                 105                 110

Lys Glu Glu Val Ile Lys Arg Lys Ile Pro Tyr Ile Leu
        115                 120                 125

<210>  8
<211>  21
<212>  PRT
<213>  Homo sapiens

<400>  8

Lys Ile Pro Tyr Ile Leu Lys Arg Gln Leu Tyr Glu Asn Lys Pro Arg
1               5                   10                  15

Arg Pro Tyr Ile Leu
            20

<210>  9
<211>  28
<212>  PRT
<213>  Homo sapiens

<400>  9

Lys Ile Pro Tyr Ile Leu Lys Arg Gln Leu Tyr Glu Asn Lys Pro Arg
1               5                   10                  15

Arg Pro Tyr Ile Leu Lys Arg Asp Ser Tyr Tyr Tyr
            20                  25

<210>  10
<211>  20
<212>  PRT
<213>  Homo sapiens

<400>  10

Gln Leu Tyr Glu Asn Lys Pro Arg Arg Pro Tyr Ile Leu Lys Arg Asp
1               5                   10                  15

Ser Tyr Tyr Tyr
            20

<210>  11
<211>  243
<212>  PRT
<213>  Homo sapiens

<400>  11

Glu Cys Ser Gln Asp Cys Ala Thr Cys Ser Tyr Arg Leu Val Arg Pro
1               5                   10                  15

Ala Asp Ile Asn Phe Leu Ala Cys Val Met Glu Cys Glu Gly Lys Leu
            20                  25                  30

Pro Ser Leu Lys Ile Trp Glu Thr Cys Lys Glu Leu Leu Gln Leu Ser
        35                  40                  45

Lys Pro Glu Leu Pro Gln Asp Gly Thr Ser Thr Leu Arg Glu Asn Ser
    50                  55                  60

Lys Pro Glu Glu Ser His Leu Leu Ala Lys Arg Tyr Gly Gly Phe Met
65                  70                  75                  80

Lys Arg Tyr Gly Gly Phe Met Lys Lys Met Asp Glu Leu Tyr Pro Met

                85                  90                  95

Glu Pro Glu Glu Glu Ala Asn Gly Ser Glu Ile Leu Ala Lys Arg Tyr
            100                 105                 110

Gly Gly Phe Met Lys Lys Asp Ala Glu Glu Asp Asp Ser Leu Ala Asn
        115                 120                 125

Ser Ser Asp Leu Leu Lys Glu Leu Leu Glu Thr Gly Asp Asn Arg Glu
    130                 135                 140

Arg Ser His His Gln Asp Gly Ser Asp Asn Glu Glu Glu Val Ser Lys
145                 150                 155                 160

Arg Tyr Gly Gly Phe Met Arg Gly Leu Lys Arg Ser Pro Gln Leu Glu
                165                 170                 175

Asp Glu Ala Lys Glu Leu Gln Lys Arg Tyr Gly Gly Phe Met Arg Arg
            180                 185                 190

Val Gly Arg Pro Glu Trp Trp Met Asp Tyr Gln Lys Arg Tyr Gly Gly
        195                 200                 205

Phe Leu Lys Arg Phe Ala Glu Ala Leu Pro Ser Asp Glu Glu Gly Glu
    210                 215                 220

Ser Tyr Ser Lys Glu Val Pro Glu Met Glu Lys Arg Tyr Gly Gly Phe
225                 230                 235                 240

Met Arg Phe

<210> 12
<211> 73
<212> PRT
<213> Homo sapiens

<400> 12

Glu Cys Ser Gln Asp Cys Ala Thr Cys Ser Tyr Arg Leu Val Arg Pro
1               5                   10                  15

Ala Asp Ile Asn Phe Leu Ala Cys Val Met Glu Cys Glu Gly Lys Leu
            20                  25                  30

Pro Ser Leu Lys Ile Trp Glu Thr Cys Lys Glu Leu Leu Gln Leu Ser
        35                  40                  45

Lys Pro Glu Leu Pro Gln Asp Gly Thr Ser Thr Leu Arg Glu Asn Ser

50                  55                  60

Lys Pro Glu Glu Ser His Leu Leu Ala
65                  70

<210>  13
<211>  5
<212>  PRT
<213>  Homo sapiens

<400>  13

Tyr Gly Gly Phe Met
1               5

<210>  14
<211>  5
<212>  PRT
<213>  Homo sapiens

<400>  14

Tyr Gly Gly Phe Leu
1               5

<210>  15
<211>  20
<212>  PRT
<213>  Homo sapiens

<400>  15

Met Asp Glu Leu Tyr Pro Met Glu Pro Glu Glu Glu Ala Asn Gly Ser
1               5                   10                  15

Glu Ile Leu Ala
            20

<210>  16
<211>  41
<212>  PRT
<213>  Homo sapiens

<400>  16

Asp Ala Glu Glu Asp Asp Ser Leu Ala Asn Ser Ser Asp Leu Leu Lys
1               5                   10                  15

Glu Leu Leu Glu Thr Gly Asp Asn Arg Glu Arg Ser His His Gln Asp
            20                  25                  30

Gly Ser Asp Asn Glu Glu Glu Val Ser
        35                  40

<210>  17
<211>  8
<212>  PRT
<213>  Homo sapiens

<400>  17

Tyr Gly Gly Phe Met Arg Gly Leu
1               5


<210>  18
<211>  12
<212>  PRT
<213>  Homo sapiens

<400>  18

Ser Pro Gln Leu Glu Asp Glu Ala Lys Glu Leu Gln
1               5                   10


<210>  19
<211>  11
<212>  PRT
<213>  Homo sapiens

<400>  19

Val Gly Arg Pro Glu Trp Trp Met Asp Tyr Gln
1               5                   10


<210>  20
<211>  22
<212>  PRT
<213>  Homo sapiens

<400>  20

Phe Ala Glu Ala Leu Pro Ser Asp Glu Glu Gly Glu Ser Tyr Ser Lys
1               5                   10                  15


Glu Val Pro Glu Met Glu
            20


<210>  21
<211>  29
<212>  PRT
<213>  Homo sapiens

<400>  21

Phe Ala Glu Ala Leu Pro Ser Asp Glu Glu Gly Glu Ser Tyr Ser Lys
1               5                   10                  15


Glu Val Pro Glu Met Glu Lys Arg Tyr Gly Gly Phe Met
            20                  25

<210>  22
<211>  7
<212>  PRT
<213>  Homo sapiens

<400>  22

Tyr Gly Gly Phe Met Arg Phe
1               5


<210>  23
<211>  23
<212>  PRT
<213>  Homo sapiens

<400>  23

His Cys Ser Asp Ser Glu Glu Glu Met Lys Ala Leu Glu Ala Asp Phe
1               5                   10                  15


Leu Thr Asn Met His Asn His
            20


<210>  24
<211>  23
<212>  PRT
<213>  Homo sapiens

<400>  24

His Cys Asn Leu Asn Ser Pro Ala Glu Glu Thr Gly Glu Val His Glu
1               5                   10                  15


Glu Glu Leu Val Ala Asn His
            20


<210>  25
<211>  6
<212>  PRT
<213>  Homo sapiens

<400>  25

Asp Ala Glu Glu Asp Asp
1               5


<210>  26
<211>  14
<212>  PRT
<213>  Homo sapiens

<400>  26

Glu Glu Asp Asp Ser Leu Ala Asn Ser Ser Asp Leu Leu Lys
1               5                   10

<210>  27
<211>  8
<212>  PRT
<213>  Homo sapiens

<400>  27

Leu Lys Glu Leu Leu Glu Thr Gly
1               5


<210>  28
<211>  17
<212>  PRT
<213>  Homo sapiens

<400>  28

Thr Gly Asp Asn Arg Glu Arg Ser His His Gln Asp Gly Ser Asp Asn
1               5                   10                  15


Glu


<210>  29
<211>  8
<212>  PRT
<213>  Homo sapiens

<400>  29

Ser Asp Asn Glu Glu Glu Val Ser
1               5

## Description

Subject matter of the present invention is a method for stratifying a female subject for hormone replacement therapy.

Vasomotor symptoms are a prominent feature of the menopause transition, affecting more than 70% of women and disturbing sleep and diminishing quality of life in 15%-20% (Bassuk SS, Manson JE. Menopausal hormone therapy and cardiovascular disease risk: Utility of biomarkers and clinical factors for risk stratification. Clinical chemistry 2014;60:68-77). Hormone replacement therapy (HRT; the main types of hormones involved are estrogens, progesterone or progestogen) is by far the most effective treatment for such symptoms. It is also indicated to prevent osteoporosis in postmenopausal women. Since randomized clinical trials have shown an unfavorable balance of benefits and risks for many women (Santen RJ, Allred DC, Ardoin SP, Archer DF, Boyd N, Braunstein GD, et al. Postmenopausal hormone therapy: An endocrine society scientific statement. The Journal of clinical endocrinology and metabolism 2010;95:s1-s66), its use has sharply declined in recent years (Sprague BL, Trentham-Dietz A, Cronin KA. A sustained decline in postmenopausal hormone use: Results from the national health and nutrition examination survey, 1999-2010. Obstetrics and gynecology 2012;120:595-603).

HRT has been shown to be associated with an increased risk for development of several types of incident adverse events, such as breast cancer (Chlebowski RT, Hendrix SL, Langer RD, Stefanick ML, Gass M, Lane D, et al. Influence of estrogen plus progestin on breast cancer and mammography in healthy postmenopausal women: The women's health initiative randomized trial. JAMA : the journal of the American Medical Association 2003;289:3243-53 - Chlebowski RT, Manson JE, Anderson GL, Cauley JA, Aragaki AK, Stefanick ML, et al. Estrogen plus progestin and breast cancer incidence and mortality in the women's health initiative observational study. Journal of the National Cancer Institute 2013;105:526-35-Beral V, Million Women Study C. Breast cancer and hormone-replacement therapy in the million women study. Lancet 2003;362:419-27), endometrial cancer (Grady D, Gebretsadik T, Kerlikowske K, Ernster V, Petitti D. Hormone replacement therapy and endometrial cancer risk: A meta-analysis. Obstetrics and gynecology 1995;85:304-13), venothromboembolism (VTE; pulmonary embolism or deep vein thrombosis) (Rachon D, Teede H. Postmenopausal hormone therapy and the risk of venous thromboembolism. Climacteric : the journal of the International Menopause Society 2008;11:273-9), coronary heart disease (dependent on age and years since menopause) (Santen RJ, Allred DC, Ardoin SP, Archer DF, Boyd N, Braunstein GD, et al. Postmenopausal hormone therapy: An endocrine society scientific statement. The Journal of clinical endocrinology and metabolism 2010;95:s1-s66 - Manson JE, Hsia J, Johnson KC, Rossouw JE, Assaf AR, Lasser NL, et al. Estrogen plus progestin and the risk of coronary heart disease. The New England journal of medicine 2003;349:523-34-Rossouw JE, Prentice RL, Manson JE, Wu L, Barad D, Barnabei VM, et al. Postmenopausal hormone therapy and risk of cardiovascular disease by age and years since menopause. JAMA : the journal of the American Medical Association 2007;297:1465-77), stroke (Wassertheil-Smoller S, Hendrix SL, Limacher M, Heiss G, Kooperberg C, Baird A, et al. Effect of estrogen plus progestin on stroke in postmenopausal women: The women's health initiative: A randomized trial. JAMA : the journal of the American Medical Association 2003;289:2673-84 - Hendrix SL, Wassertheil-Smoller S, Johnson KC, Howard BV, Kooperberg C, Rossouw JE, et al. Effects of conjugated equine estrogen on stroke in the women's health initiative. Circulation 2006;113:2425-34), and potentially others.

Benefit/risk profiles might differ depending on the type and composition of hormonal products used in HRT as well as their routes of application (Sturdee DW, Pines A, International Menopause Society Writing G, Archer DF, Baber RJ, Barlow D, et al. Updated ims recommendations on postmenopausal hormone therapy and preventive strategies for midlife health. Climacteric : the journal of the International Menopause Society 2011;14:302-20). However, the evidence demonstrating this is limited.

Current guidelines concerning the use of HRT are relatively vague and difficult to apply individually. The recommendations of the International Menopause Society (IMS) say (Sturdee DW, Pines A, International Menopause Society Writing G, Archer DF, Baber RJ, Barlow D, et al. Updated ims recommendations on postmenopausal hormone therapy and preventive strategies for midlife health. Climacteric : the journal of the International Menopause Society 2011;14:302-20): *"Consideration of HRT should be part of an overall strategy including lifestyle recommendations regarding diet, exercise, smoking cessation and safe levels of alcohol consumption for maintaining the health of peri- and postmenopausal women. HRT must be individualized and tailored according to symptoms and the need for prevention, as well as personal and family history, results of relevant investigations, the woman's preferences and expectations. The risks and benefits of HRT differ for women during the menopause transition compared to those for older women."*

It would be most helpful, if there was a way to identify women, for whom benefits will outweigh the risks, but this is currently considered to remain a challenge (Bassuk SS, Manson JE. Menopausal hormone therapy and cardiovascular disease risk: Utility of biomarkers and clinical factors for risk stratification. Clinical chemistry 2014;60:68-77).

Recently, it has been suggested that it may be possible to classify women as better or worse candidates for HRT by using individual risk stratification (Bassuk SS, Manson JE. Menopausal hormone therapy and cardiovascular disease risk: Utility of biomarkers and clinical factors for risk stratification. Clinical chemistry 2014;60:68-77). In the development of a personalized approach to the prediction of risk for cardiovascular disease events for women while on HRT, clinical characteristics, serum biomarkers, genomic markers, and gene-environment interactions have been analyzed: The available data suggest several characteristics of women who are optimal candidates for HRT use: younger age (<60 years), recent onset of menopause (<10 years), favorable lipid profile (LDL cholesterol <130 mg/dL or LDL/HDL cholesterol ratio <2.5), absence of metabolic syndrome, and absence of factor V Leiden genotype (Bassuk SS, Manson JE. Menopausal hormone therapy and cardiovascular disease risk: Utility of biomarkers and clinical factors for risk stratification. Clinical chemistry 2014;60:68-77).

Also, factors have been identified which influence the HRT-associated risk for the development of breast cancer: In women with low/normal BMI and extremely dense breasts, HRT use was associated with the highest breast cancer risk, compared with non-users. In overweight/obese women with less-dense breasts, no excess risk was associated with HRT use (Hou N, Hong S, Wang W, Olopade OI, Dignam JJ, Huo D. Hormone replacement therapy and breast cancer: Heterogeneous risks by race, weight, and breast density. Journal of the National Cancer Institute 2013;105:1365-72). Black women, as opposed to white, Asian and Hispanic women, did not have an increased risk (Hou N, Hong S, Wang W, Olopade OI, Dignam JJ, Huo D. Hormone replacement therapy and breast cancer: Heterogeneous risks by race, weight, and breast density. Journal of the National Cancer Institute 2013;105:1365-72). Women initiating HRT closer to menopause had higher breast cancer risk with linear diminishing influence as time from menopause increased (Chlebowski RT, Manson JE, Anderson GL, Cauley JA, Aragaki AK, Stefanick ML, et al. Estrogen plus progestin and breast cancer incidence and mortality in the women's health initiative observational study. Journal of the National Cancer Institute 2013;105:526-35 - Beral V, Reeves G, Bull D, Green J, Million Women Study C. Breast cancer risk in relation to the interval between menopause and starting hormone therapy. Journal of the National Cancer Institute 2011;103:296-305).

Plasma concentrations of Pro-Neurotensin and Pro-Enkephalin are known to be associated with the risk of women in the general population to develop incident breast cancer (WO2014/053502; WO2013/132089), but how these markers perform in women - especially postmenopausal women - under HRT is not known. Surprisingly, plasma concentrations of Pro-Neurotensin and Pro-Enkephalin are excellent stratification marker for HRT.

Subject matter of the present invention is a method for stratifying a female subject for hormone replacement therapy comprising:
- determining the level of Pro-Neurotensin or fragments thereof of at least 5 amino acids in a bodily fluid obtained from said female subject; and
- comparing said level of Pro-Neurotensin or fragments thereof with a pre-determined pro-NT threshold and
- wherein in case the determined level of Pro-Neurotensin and fragments thereof is above said pre-determined threshold then the subject is identified as having an enhanced risk to suffer an adverse event or having an enhanced risk to attract a disease in case of a hormone replacement therapy, and
- wherein in case the determined level of Pro-Neurotensin and fragments thereof is below said pre-determined threshold then the subject is identified as not having an enhanced risk to suffer an adverse event or as not having an enhanced risk to attract a disease in case of a hormone replacement therapy, and/or
- determining the level of Pro-Enkephalin or fragments thereof of at least 5 amino acids (proENK) in a bodily fluid obtained from said female subject; and
- comparing said level of Pro-Enkephalin or fragments thereof with a pre-determined "proENK threshold" and
- wherein in case the determined level of Pro-Enkephalin or fragments thereof is below said pre-determined proENK threshold then the subject is identified as having an enhanced risk to suffer an adverse event or having an enhanced risk to attract a disease in case of a hormone replacement therapy, and
- wherein in case the determined level of Pro-Enkephalin or fragments thereof is above said pre-determined pENK threshold then the subject is identified as not having an enhanced risk to suffer an adverse event or as not having an enhanced risk to attract a disease in case of a hormone replacement therapy .

Stratification is defined as the identification of a group of patients with shared "biological" characteristics by using molecular, biochemical or imaging diagnostic testing to select the optimal management for the patients and achieve the best possible outcome in terms of (based on the category and disease characteristics): Risk assessment and prevention, Achievement of the optimal treatment outcome (http://ec.europa.eu/research/health/pdf/biomarkers-for-patient-stratification_en.pdf). Such stratification may occur prior to initiating a therapy and/or during the therapy.

In one specific embodiment said adverse event or disease is selected from the group comprising diabetes, metabolic syndrome, cardiac event, cardiac disease, and cancer, in particular breast cancer or lung cancer. Examples of cancers may be selected from the group comprising breast cancer, lung cancer, pancreatic cancer and colon cancer.

The term "subject" as used herein refers to a living human or non-human organism. Preferably, herein the subject is a human subject.

The term "enhanced level" means a level above a certain threshold level.

The term "reduced level" means a level below a certain threshold level.

A bodily fluid may be selected from the group comprising blood, serum, plasma, urine, cerebro spinal liquid (csf), and saliva.

In one specific embodiment of the invention said female subject is either going through menopause or is post-menopausal.

In one specific embodiment of the invention said subject does not suffer from diabetes. In one specific embodiment of the invention said subject does not suffer from metabolic syndrome. In one specific embodiment of the invention said subject does have not suffered a cardiac event. In one specific embodiment of the invention said subject does have not suffered a cardiac disease. In one specific embodiment said subject does not suffer from cancer, in particular breast cancer, lung cancer, pancreatic cancer or colon cancer.

In one specific embodiment of the invention said female subject has not yet received hormone replacement therapy. In another specific embodiment of the invention said female subject is receiving hormone placement therapy.

In one specific embodiment of the invention wherein in case the level of Pro-Neurotensin or fragments thereof is higher than said pre-determined pro-NT threshold and / or if the level of Pro-Enkephalin or fragments thereof of at least 5 amino acids is lower than said pre-determined pENK threshold then hormone replacement therapy is either withhold from said female subject or hormone replacement therapy will be stopped in said female subject.

In one specific embodiment Pro-Neurotensin or fragments thereof is selected from Pro-Neurotensin 1-117, fragments of Pro-Neurotensin 1-117 (SEQ ID NO: 5) of at least 5 amino acids or Pro-Neurotensin 1-117 (SEQ ID NO: 5) comprising peptides.

Fragments of Pro-Neurotensin that may be determined in a bodily fluid may be e.g. selected from the group of the following fragments:
SEQ ID NO: 1 (Pro-Neurotensin 1-147) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYILKRQLY ENKPRRPYIL KRDSYYY
SEQ ID NO: 2 (Pro-Neurotensin 1-125 (large neuromedin N)) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVI KR KIPYIL
SEQ ID NO: 3 (neuromedin N:)
   KIPYIL
SEQ ID NO: 4 (neurotensin)
   pyroQLYENKPRRP YIL
SEQ ID NO: 5 (Pro-Neurotensin 1-117) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVI
SEQ ID NO: 6 (Pro-Neurotensin 1-132) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYILKRQLY EN
Seq ID No 7: (Pro-Neurotensin 1-125) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYIL
SEQ ID NO: 8 (Pro-Neurotensin 120-140)
   KIPYILKRQL YENKPRRPYI L
SEQ ID NO: 9 (Pro-Neurotensin 120-147)
   KIPYILKRQL YENKPRRPYIL KRDSYYY
SEQ ID NO: 10 (Pro-Neurotensin 128-147)
   QLYENKPRRP YILKRDSYYY

In a more specific embodiment of the method according to the present invention the level of Pro-Neurotensin 1-117 (SEQ ID NO: 5) and/or fragments thereof is determined.

Determining the level of Pro-Neurotensin or fragments thereof may mean that the immunoreactivity towards pro-neurotensin or fragments thereof is determined. A binder used for determination of Pro-Neurotensin or fragments thereof depending of the region of binding may bind to more than one of the above displayed molecules. This is clear to a person skilled in the art.

Thus, according to the present invention the level of immunoreactive analyte by using at least one binder that binds to a region within the amino acid sequence of any of the above peptide and peptide fragments, (i.e. Pro-Neurotensin and fragments according to any of the sequences 1 to 10), is determined in a bodily fluid obtained from said subject; and correlated to the specific embodiments of clinical relevance.

In a specific embodiment the level of Pro-Neurotensin is measured with an immunoassay. More specifically an immunoassay is used as described in Ernst et al. (Peptides (2006), (27) 1787-1793). An immunoassay that may be useful for determining the level of Pro-Neurotensin or fragments thereof of at least 5 amino acids may comprise the steps as outlined in Example 2. All thresholds and values have to be seen in correlation to the test and the calibration used according to Example 2. A person skilled in the art may know that the absolute value of a threshold might be influenced by the calibration used. This means that all values and thresholds given herein are to be understood in context of the calibration used in herein (Example 2). A human pro-NT-calibrator is available by ICI-Diagnostics, Berlin, Germany. Alternatively, the assay may also be calibrated by synthetic or recombinant P-NT 1-117 (SEQ ID NO: 5) or fragments thereof (see also Ernst et. al, 2006).

The pre-determined threshold for determining the risk in a female subject according to the methods of the present invention is above 78 pmol/l PNT, preferred 100 pmol/1, more preferred 130 pmol/1, more preferred 150 pmol/l more preferred 160 pmol/1, more preferred 190 pmol/1. In a specific embodiment said threshold is about 100 pmol/1. In another embodiment about 130 pmol/1, more preferred about 160 pmol/1, more preferred about 190 pmol/1. These thresholds are related to the above mentioned calibration method. A pro-NT value above said threshold means that the subject has an enhanced risk of getting cancer.

Throughout the specification pre-determined pro-NT threshold means the threshold for the level of Pro-Neurotensin and/or fragments thereof. Throughout the specification pre-determined pENK encompasses the level of Pro-Enkephalin and/or fragments thereof.

In one specific embodiment Pro-Enkephalin or fragments thereof is selected from MRPENK (MRPENK is SEQ ID NO. 16: Pro-Enkephalin 119-159, Mid regional Pro-Enkephalin-fragment, MRPENK). Fragments of MRPENK of at least 5 amino acids or MRPENK comprising peptides.

Pro-Enkephalin has the following sequence:
SEQ ID NO. 11 (Pro-Enkephalin (1-243)

ECSQDCATCSYRLVRPADINFLACVMECEGKLPSLKIWETCKELLQLSKPELPQDGTS
TL
RENSKPEESHLLAKRYGGFMKRYGGFMKKMDELYPMEPEEEANGSEILAKRYGGFM
KKDAEEDDSLANSSDLLKELLETGDNRERSHHQDGSDNEEEVSKRYGGFMRGLKRS
PQLEDEAKELQKRYGGFMRRVGRPEWWMDYQKRYGGFLKRFAEALPSDEEGESYS
KEVPEMEKRYGGF MRF

Fragments of Pro-Enkephalin that may be determined in a bodily fluid may be *e.g.* selected from the group of the following fragments:
SEQ ID NO. 12 (Syn-Enkephalin, Pro-Enkephalin 1-73) 
ECSQDCATCSYRLVRPADINFLACVMECEGKLPSLKIWETCKELLQLSKPELPQDGTS
TL
RENSKPEESHLLA
SEQ ID NO. 13 (Met-Enkephalin)
   YGGFM
SEQ ID NO. 14 (Leu-Enkephalin)
   YGGFL
SEQ ID NO. 15 (Pro-Enkephalin 90-109)
   MDELYPMEPEEEANGSEILA
SEQ ID NO. 16 (Pro-Enkephalin 119-159, Mid regional Pro-Enkephalin-fragment, MRPENK)
   DAEEDDSLANSSDLLKELLETGDNRERSHHQDGSDNEEEVS
SEQ ID NO. 17 (Met-Enkephalin-Arg-Gly-Leu)
   YGGFMRGL
SEQ ID NO. 18 (Pro-Enkephalin 172-183)
   SPQLEDEAKELQ
SEQ ID NO. 19 (Pro-Enkephalin 193-203)
   VGRPEWWMDYQ
SEQ ID NO. 20 (Pro-Enkephalin 213-234)
   FAEALPSDEEGESYSKEVPEME
SEQ ID NO. 21 (Pro-Enkephalin 213-241)
   FAEALPSDEEGESYSKEVPEMEKRYGGF M
SEQ ID NO. 22 (Met-Enkephalin-Arg-Phe)
   YGGFMRF

Determining the level of Pro-Enkephalin including Leu-Enkephalin and Met-Enkephalin or fragments thereof may mean that the immunoreactivity towards Pro-Enkephalin or fragments thereof including Leu-Enkephalin and Met-Enkephalin is determined. A binder used for determination of Pro-Enkephalin including Leu-Enkephalin and Met-Enkephalin or fragments thereof depending of the region of binding may bind to more than one of the above displayed molecules. This is clear to a person skilled in the art.

Thus, according to the present invention the level of immunoreactive analyte by using at least one binder that binds to a region within the amino acid sequence of any of the above peptide and peptide fragments, (i.e. Pro-Enkephalin (pENK) and fragments according to any of the sequences 1 to 12), is determined in a bodily fluid obtained from said subject; and correlated to the specific embodiments of clinical relevance.

In a more specific embodiment of the method according to the present invention the level of MRPENK is determined (SEQ ID NO. 16: Pro-Enkephalin SE, Mid regional Pro-Enkephalin-fragment, MRPENK). In a more specific embodiment the level of immunoreactive analyte by using at least one binder that binds to MRPENK is determined and is correlated to the specific embodiments of clinical relevance according to the invention.

Determining the level of Pro-Enkephalin or fragments thereof including Leu-Enkephalin and Met-Enkephalin or fragments thereof may mean that the immunoreactivity towards Pro-Enkephalin or fragments thereof including Leu-Enkephalin and Met-Enkephalin is determined. A binder used for determination of Pro-Enkephalin including Leu-Enkephalin and Met-Enkephalin or fragments thereof depending of the region of binding may bind to more than one of the above displayed molecules. This is clear to a person skilled in the art. In another embodiment of the invention the fragment is not Leu-Enkephalin or Met-Enkephalin, In another embodiment of the invention the immunoreactivity towards Pro-Enkephalin or fragments thereof not including Leu-Enkephalin and Met-Enkephalin is determined.

In a more specific embodiment of the method according to the present invention the level of MRPENK. (SEQ ID NO. 16 (Pro-Enkephalin 119-159, Mid regional Pro-Enkephalin-fragment, MRPENK, DAEEDDSLANSSDLLKELLETGDNRERSHHQDGSDNEEEVS) is determined.

In one embodiment of the present invention the level of Pro-Neurotensin or fragments thereof and / or the level of Pro-Enkephalin or fragments thereof of at least 5 amino acids is measured with an immunoassay.

In a specific embodiment the level of Pro-Neurotensin or fragments thereof and / or the level of Pro-Enkephalin or fragments thereof of at least 5 amino acids are measured with an immunoassay using antibodies or fragments of antibodies binding to Pro-Enkephalin or fragments thereof and/or antibodies or fragments of antibodies binding to pro-neurotensin or fragments thereof. Immunoassays that may be useful in this regard may comprise the steps as outlined in Example 3. All thresholds and values have to be seen in correlation to the test and the calibration used according to Example 3. A person skilled in the art may know that the absolute value of a threshold might be influenced by the calibration used. This means that all values and thresholds given herein are to be understood in context of the calibration used in herein (Example 3).

In addition to antibodies other biopolymer scaffolds are well known in the art to complex a target molecule and have been used for the generation of highly target specific biopolymers.

Non-Ig scaffolds may be protein scaffolds and may be used as antibody mimics as they are capable to bind to ligands or antigenes. Non-Ig scaffolds may be selected from the group comprising tetranectin-based non-Ig scaffolds (e.g. described in US2010/0028995), fibronectin scaffolds (e.g. described in EP 1 266 025, lipocalin-based scaffolds ((e.g. described in WO 2011/154420); ubiquitin scaffolds (e.g. described in WO2011/073214) transferring scaffolds (e.g. descripted in US 2004/0023334), protein A scaffolds (e.g. descripted in EP 2 231 860), ankyrin repeat based scaffolds (e.g. descripted in WO 2010/060748), microprotein, preferably microproteins forming a cysteine knot) scaffolds (e.g. described in EP 2 314 308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1 941 867).

For instance, in the United States Patent application 20100298541 (http://www.freepatentonline.com/y2010/0298541.html) it is said in section 6, detailed description: "Moreover, the scaffold of the invention provide the functional advantages of antibody molecules. In particular, despite the fact that the scaffold is not an immunoglobin, its overall fold is closed to that of the variable region of the IgG heavy chain, making it possible to display its three loops in an analogous fashion to antibody CDRs in relative orientations. Because of this structure, the scaffolds of the invention possess antigen binding properties that are similar in nature and affinity to those of antibodies. As a result, loop randomization and shuffling strategies may be employed in vitro that are similar to the process of affinity maturation of antibodies in vivo". Thus, it is clear that scaffolds are to be considered equivalent to antibodies concerning their antigen binding properties and affinities.

This is further corroborated by many publications, only to mentioned here one a relevant review article of Gebauer et al., Engineered protein scaffolds as next-generation antibody therapeutics, Current Opinion in Chemical Biology 2009, 13:245-255, where it says: "In parallel to the increasingly advanced manipulation of Ig fragments an independent development has focused on recruiting unrelated proteins for analogous applications. In fact, it was demonstrated that several protein families with non-Ig architecture can be equipped with novel binding sites by employing methods of combinatorial engineering, such as site-directed random mutagenesis in combination with phage display or other molecular selection techniques. As result, novel biomolecular binding reagents have become available, thus triggering a paradigm shift in so far as antibodies are no longer considered as the unique and universal class of receptor proteins in biotechnology and medicine."

In another recent review of Wurch T, Pierre A, Depil S: Novel protein scaffolds as emerging therapeutic proteins: from discovery to clinical proof-of-concept. Trends in biotechnology 2012, 30(11):575-582, the abstract starts reading: "Recent advances in combinatorial protein engineering have been made possible to develop immnunoglobin (Ig)-based and non-Ig protein scaffolds that can potentially substitute for most whole antibody-associated properties and currently translate into biologicals with drug-like properties.

Binder that may be used for determining the level of Pro-Enkephalin or Pro-Neurotensin or fragments thereof exhibit an affinity constant to Pro-Enkephalin or Pro-Neurotensin or fragments thereof of at least 10⁷ M⁻¹, preferred 10⁸ M⁻¹, preferred affinity constant is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention. Binding affinity may be determined using the Biacore method, offered as service analysis e.g. at Biaffin, Kassel, Germany (http://www.biaffin.com/de/).

A human Pro-Enkephalin-control sample is available by ICI-Diagnostics, Berlin, Germany http://www.ici-diagnostics.com/. The assay may also be calibrated by synthetic (for our experiments we used synthetic MRPENK, SEQ ID NO. 16) or recombinant Pro-Enkephalin or fragments thereof.

The threshold for determining the risk in a female subject according to the methods of the present invention is below 100 pmol/l pENK, preferred below 50 pmol/l, preferred below 48 pmol/1, preferred below 46 pmol/1, preferred below 44 pmol/l, more preferred below 40,4 pmol/1. In a specific embodiment said threshold is about 40,4 pmol/l. In a specific embodiment the threshold is 44 pmol/l or 46 pmol/l or 48 pmol/l. These thresholds are related to the above mentioned calibration method. A pENK value below said threshold means that the subject has an enhanced risk of getting cancer or has already cancer.

Alternatively the level of any of the above analytes may be determined by other analytical methods e.g. mass spectroscopy.

In one embodiment the threshold may be pre-determined as follows:
- Comparison of concentration of the marker in a bodily fluid obtained from said subject with the median of the marker in a bodily fluid obtained from an ensemble of pre-determined samples in a randomly selected population of subjects having comparable baseline conditions as said subject,
- Comparison of concentration the marker in a bodily fluid obtained from said subject with an quantile of the levels of the marker, and/or its isoforms in a bodily fluid obtained from an ensemble of pre-determined samples in a population of subjects having comparable baseline conditions as said subject,
- Calculation based on Cox Proportional Hazards analysis or by using Risk index calculations such as the NRI (Net Reclassification Index) or the IDI (Integrated Discrimination Index).

The term "marker" according to the present invention throughout the specification is either pro-neurotensin or fragments thereof of at least 5 amino acids or Pro-Enkephalin or fragments thereof of at least 5 amino acids or any of the above mentioned fragments.

In one embodiment that sample is a blood sample selected from the group comprising whole blood, serum, and plasma.

In one embodiment of the invention the blood sample is a fastening sample.

Fastening sample means a sample, which has been drawn from a subject, which has not consumed any food or solubilized nutrients for at least 8 hours prior to blood sampling. In a specific embodiment, such fastening sample is taken from a subject between 7:00 and 11:30 a,m., after the subject has fastened at least 8 hours.

In another embodiment fasting levels means the marker level determined in blood, serum or plasma of fastened subjects that did not have food uptake 12 h prior sample taking. In said embodiment fastened subject means a subject that had 12 h prior sample taking no food uptake.

In one embodiment of the invention said female subject has never had a history of diagnosis of cancer at the time the sample of bodily fluid is taken from said female subject.

According to the methods of the present invention additionally at least one parameter may be determined selected from the group comprising: age, presence of diabetes mellitus, current smoking, Time since onset of menopause, LDL cholesterol, LDL/HDL cholesterol ratio, presence of metabolic syndrome, and presence of factor V Leiden genotype, body mass index, breast density, race/ethnicity.

According to the present invention it is subject matter to make a risk stratification of women for HRT:
As an example for a possible combination, three risk groups were defined as follows:
   First, "high-" and "low-risk"-groups were defined for the two markers separately:
      A Pro-Neurotensin "high risk"-group was defined as subjects with Pro-Neurotensin concentrations above 190 pmol/L, and a Pro-Neurotensin "low risk"-group was defined as subjects with Pro-Neurotensin concentrations below 190 pmol/L.

A Pro-Enkepahlin "high risk"-group was defined as subjects with Pro-Enkepahlin concentrations below 46 pmol/L, and a Pro-Enkepahlin "low risk"-group was defined as subjects with Pro- Enkepahlin concentrations above 46 pmol/L.

For the combination of both markers, a "low risk"-group was defined as subjects being in the "low risk"-group of both markers, a "medium risk"-group was defined as subjects being in the "low risk"-group of one marker and in the "high risk"-group of the other marker, and a "high risk"-group was defined as subjects being in the "high risk"-group of both markers.

For the above described stratification and assignment to risk groups other threshold may be used depending on the sensitivity and specificity the person skilled in the art would accept. Thus, the threshold for pro-neurotensin for assignment to risk groups may be also about 100 pmol/1. In another embodiment about 130 pmol/1, more preferred about 160 pmol/1, more preferred about 190 pmol/l. These thresholds are related to the above mentioned calibration method.

Thus, the threshold for Pro-Enkephalin for assignment to risk groups may be about 40,4 pmol/1. In a specific embodiment the threshold may be about 44 pmol/l or 46 pmol/l or 48 pmol/1. These thresholds are related to the above mentioned calibration method.

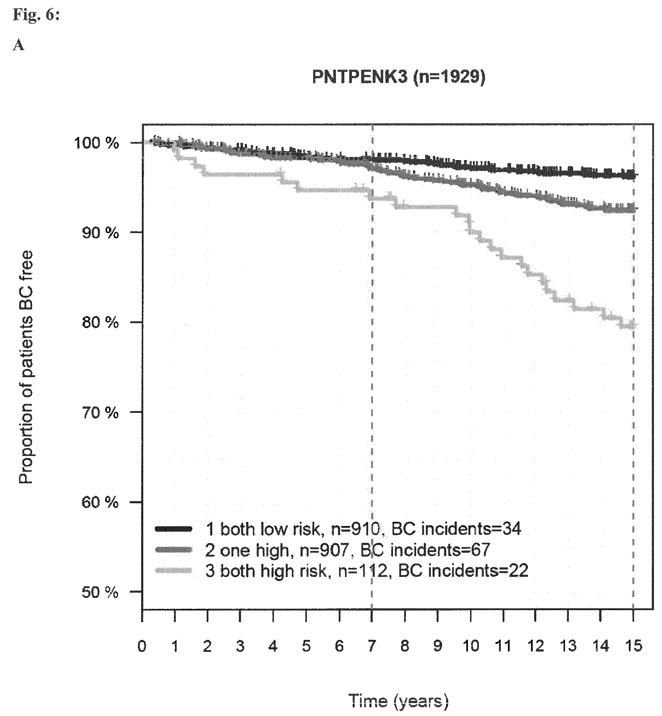

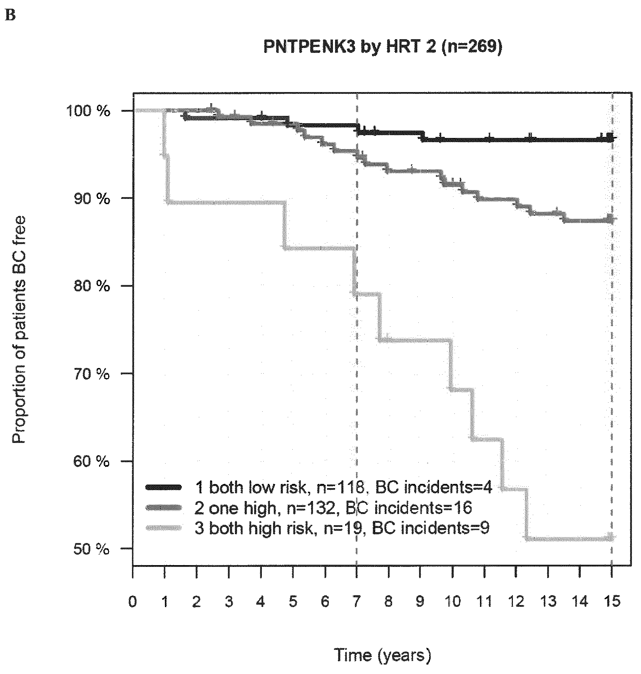

These results demonstrate that risk stratification by combining plasma pro-Neurotensin and plasma Pro-Enkephalin is three times as strong in postmenopausal women receiving HRT compared to all women. (Fig. 6 a and b).

Thus, both biomarker, pro-NT and pENK are helpful tools for stratifying women for HRT.

Embodiments are:
1) A method for stratifying a female subject for hormone replacement therapy comprising:
   - determining the level of Pro-Neurotensin or fragments thereof of at least 5 amino acids in a bodily fluid obtained from said female subject; and
   - comparing said level of Pro-Neurotensin or fragments thereof with a pre-determined "pro-NT threshold" and
   - wherein in case the determined level of Pro-Neurotensin or fragments thereof is above said pre-determined threshold then the subject is identified as having an enhanced risk to suffer an adverse event or having an enhanced risk to attract a disease in case of a hormone replacement therapy, and
   - wherein in case the determined level of Pro-Neurotensin or fragments thereof is below said pre-determined threshold then the subject is identified as not having an enhanced risk to suffer an adverse event or as not having an enhanced risk to attract a disease in case of a hormone replacement therapy, and/or
   - determining the level of Pro-Enkephalin or fragments thereof of at least 5 amino acids (pENK) in a bodily fluid obtained from said female subject; and
   - comparing said level of Pro-Enkephalin or fragments thereof with a pre-determined "pENK threshold" and
   - wherein in case the determined level of Pro-Enkephalin or fragments thereof is below said pre-determined pENK threshold then the subject is identified as having an enhanced risk to suffer an adverse event or having an enhanced risk to attract a disease in case of a hormone replacement therapy, and
   - wherein in case the determined level of Pro-Enkephalin or fragments thereof is above said pre-determined pENK threshold then the subject is identified as not having an enhanced risk to suffer an adverse event or as not having an enhanced risk to attract a disease in case of a hormone replacement therapy .
2) A method for stratifying a female subject for hormone replacement therapy according to claim 1 wherein said adverse event or disease is selected from the group comprising, diabetes, metabolic syndrome, cardiac event, cardiac disease, and cancer in particular breast cancer or lung cancer.
3) A method for stratifying a female subject for hormone replacement therapy according to claim 1 or 2 wherein said female subject is either going through menopause or is post-menopausal.
4) A method for stratifying a female subject for hormone replacement therapy according to any of claims 1 to 3, wherein said subject does not suffer from cancer, in particular breast cancer or lung cancer.
5) A method for stratifying a female subject for hormone replacement therapy according to any of claims 1 to 4, wherein said subject does not suffer from diabetes.
6) A method for stratifying a female subject for hormone replacement therapy according to any of claims 1 to 5, wherein said subject does not suffer from metabolic syndrome.
7) A method for stratifying a female subject for hormone replacement therapy according to any of claims 1 to 6, wherein said subject does not have suffered a cardiac event.
8) A method for stratifying a female subject for hormone replacement therapy according to any of claims 1 to 7, wherein said subject does not have suffered a cardiac disease.
9) A method for stratifying a female subject for hormone replacement therapy according to any of claims 1-8 wherein said female subject has not yet received hormone replacement therapy.
10) A method for stratifying a female subject for hormone replacement therapy wherein said subject does not suffer from cancer according to any of claims claim 1 or 9 wherein said female subject is receiving hormone placement therapy.
11) A method for stratifying a female subject for hormone replacement therapy wherein said subject does not suffer from cancer according to any of claims claim 1 to 10 wherein in case the level of Pro-Neurotensin or fragments thereof is higher than said pre-determined pro-NT threshold and / or if the level of Pro-Enkephalin or fragments thereof of at least 5 amino acids is lower than said pre-determined pENK threshold then hormone replacement therapy is either withhold from said female subject or hormone replacement therapy will be stopped in said female subject.
12) A method for stratifying a female subject for hormone replacement therapy wherein said subject does not suffer from cancer according to any of claims claim 1 to 11 wherein Pro-Neurotensin or fragments thereof is selected from Pro-Neurotensin 1-117, fragments of Pro-Neurotensin 1-117 (SEQ ID NO: 5) of at least 5 amino acids or Pro-Neurotensin 1-117 (SEQ ID NO: 5) comprising peptides.
13) A method for stratifying a female subject for hormone replacement therapy wherein said subject does not suffer from cancer according to any of claims claim 1 to 12 wherein Pro-Enkephalin or fragments thereof is selected from the group comprising MRPENK (MRPENK is SEQ ID NO. 16: Pro-Enkephalin 119-159, Mid regional Pro-Enkephalin-fragment, MRPENK). Fragments of MRPENK of at least 5 amino acids or MRPENK comprising peptides.
14) A method according to any of claims 1 to 13, wherein the level of Pro-Neurotensin or fragments thereof of at least 5 amino acids and / or the level of Pro-Enkephalin or fragments thereof of at least 5 amino acids comprising peptides in a bodily fluid is the fasting level.
15) A method according to any of claims 1 to 13, wherein said female subject has never had a history of diagnosis of cancer at the time the sample of bodily fluid is taken from said female subject.
16) A method according to any of claims 1 to 15, wherein additionally at least one parameter is determined selected from the group comprising: age, presence of diabetes mellitus, current smoking, Time since onset of menopause, LDL cholesterol, LDL/HDL cholesterol ratio, presence of metabolic syndrome, and presence of factor V Leiden genotype, body mass index, breast density, race/ethnicity.
17) A method according to any of the preceding claims, wherein the level of MR-Pro-Enkephalin (SEQ ID No. 16) and/or Pro-Neurotensin 1-117 (SEQ ID NO: 5) is determined.
18) A method according to any of the preceding claims, wherein the level of Pro-Neurotensin or fragments thereof and / or the level of Pro-Enkephalin or fragments thereof of at least 5 amino acids is measured with an immunoassay.

### Examples

### Example 1

### Development of Antibodies

### Peptides/ conjugates for Immunization:

Peptides for immunization were synthesized (JPT Technologies, Berlin, Germany) with an additional N-terminal Cystein residue for conjugation of the peptides to bovine serum albumin (BSA). The peptides were covalently linked to BSA by using SulfoLink-Coupling gel (Perbio-science, Bonn, Germany). The coupling procedure was performed according to the manual of Perbio.

Labelled antibody (LA) peptide (P-NT 1-19):
H-CSDSEEEMKALEADFLTNMH-NH2 (SEQ ID NO: 23)

Solid phase antibody (SPA) peptide (P-NT 44-62):
H-CNLNSPAEETGEVHEEELVA-NH2 (SEQ ID NO: 24)

The antibodies were generated according to the following method:
A BALB/c mouse were immunized with 100 µg peptide-BSA-conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitonal and one intra venous injection.

Splenocytes from the immunized mouse and cells of the myeloma cell line SP2/0 were fused with 1 ml 50 % polyethylene glycol for 30 s at 37 °C. After washing, the cells were seeded in 96-well cell culture plates. Hybrid clones were selected by growing in HAT medium [RPMI 1640 culture medium supplemented with 20 % fetal calf serum and HAT-supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined.
(Lane, R.D. "A short-duration polyethylene glycol fusiontechnique for increasing production of monoclonal antibody-secreting hybridomas", J. Immunol. Meth. 81: 223-228; (1985), Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)).

### Monoclonal antibody production

Antibodies were produced via standard antibody production methods (Marx et al., Monoclonal Antibody Production (1997), ATLA 25, 121) and purified via Protein A-chromatography. The antibody purities were > 95 % based on SDS gel electrophoresis analysis.

### Example 2

### Immunoassay for the quantification of human Pro-Neurotensin

The technology used was a sandwich coated tube luminescence immunoassay, based on Acridinium ester labelling.

Labelled compound (tracer): 100 µg (100 µl) LA (1 mg/ml in PBS, pH 7.4, was mixed with 10 µl Acridinium NHS-ester (1 mg/ml in acetonitrile, InVent GmbH, Germany) (EP 0353971) and incubated for 20 min at room temperature. Labelled LA was purified by gel-filtration HPLC on Bio-Sil SEC 400-5 (Bio-Rad Laboratories, Inc., USA) The purified LA was diluted in (300 mmol/l potassiumphosphate, 100 mmol/l NaCl, 10 mmol/l Na-EDTA, 5 g/l bovine serum albumin, pH 7.0). The final concentration was approx. 800.000 relative light units (RLU) of labelled compound (approx. 20 ng labeled antibody) per 200 µl. Acridiniumester chemiluninescence was measured by using an AutoLumat LB 953 (Berthold Technologies GmbH & Co. KG).

Solid phase: Polystyrene tubes (Greiner Bio-One International AG, Austria) were coated (18 h at room temperature) with SPA (1.5 µg SPA/0.3 ml 100 mmol/l NaCl, 50 mmol/l Tris/HCl, pH 7.8). After blocking with 5 % bovine serum albumin, the tubes were washed with PBS, pH 7.4 and vacuum dried.

### Calibration:

The assay was calibrated, using dilutions of pro-NT-containing human serum. A pool of human sera with high pro-NT-immunoreactivity (InVent Diagostika, Hennigsdorf, Germany) was diluted with horse serum (Biochrom AG, Deutschland) (assay standards).

The standards were calibrated by use of the human pro-NT-calibrator (ICI-Diagnostics, Berlin, Germany). Alternatively, the assay may be calibrated by synthetic or recombinant pro-NT 1-117 (SEQ ID NO: 5) or fragments thereof (see also Ernst *et al.,* 2006).

### Pro-NT Immunoassay:

50 µl of sample (or calibrator) was pipetted into SPA coated tubes, after adding labeled LA (200ul), the tubes were incubated for 16-22 h at 18-25 °C. Unbound tracer was removed by washing 5 times (each 1 ml) with washing solution (20 mmol/l PBS, pH 7.4, 0.1 % Triton X-100).

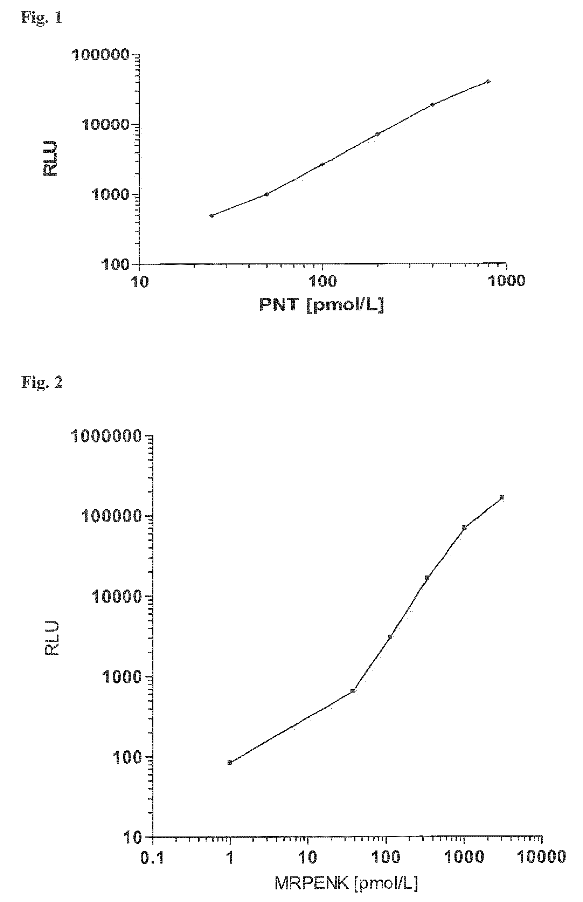

### Tube-bound LA was measured by using the LB 953. (Fig. 1)

### Example 3

### Development of Antibodies

### Peptides

Peptides were synthesized (JPT Technologies, Berlin, Germany).

### Peptides/ conjugates for Immunization:

Peptides for immunization were synthesized (JPT Technologies, Berlin, Germany) with an additional N-terminal Cystein residue for conjugation of the peptides to bovine serum albumin (BSA). The peptides were covalently linked to BSA by using Sulfo-SMCC (Perbio-science, Bonn, Germany). The coupling procedure was performed according to the manual of Perbio.

**Table 1:**

| Peptide for immunization | Pro-Enkephalin sequence |
|---|---|
| (C)DAEEDD | 119-125 (SEQ ID NO: 25) |
| (C)EEDDSLANSSDLLK | 121-134 (SEQ ID NO: 26) |
| (C)LKELLETG | 133-140 (SEQ ID NO: 27) |
| (C)TGDNRERSHHQDGSDNE | 139-155 (SEQ ID NO: 28) |
| (C)SDNEEEVS | 152-159 (SEQ ID NO: 29) |

The antibodies were generated according to the following method:
A BALB/c mouse was immunized with 100 µg peptide-BSA-conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitonal and one intravenous injection.

Spenocytes from the immunized mouse and cells of the myeloma cell line SP2/0 were fused with 1 ml 50 % polyethylene glycol for 30 s at 37 °C. After washing, the cells were seeded in 96-well cell culture plates. Hybrid clones were selected by growing in HAT medium [RPMI 1640 culture medium supplemented with 20 % fetal calf serum and HAT-supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined.

(Lane, R.D. "A short-duration polyethylene glycol fusiontechnique for increasing production of monoclonal antibody-secreting hybridomas", J. Immunol. Meth. 81: 223-228; (1985), Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)).

### Monoclonal antibody production

Antibodies were produced via standard antibody production methods (Marx et al., Monoclonal Antibody Production (1997), ATLA 25, 121) and purified via Protein A-chromatography. The antibody purities were > 95 % based on SDS gel electrophoresis analysis.

### Labelling and coating of antibodies.

All antibodies were labelled with acridinium ester according the following procedure:
Labelled compound (tracer): 100 µg (100 µl) antibody (1 mg/ml in PBS, pH 7.4, was mixed with 10 µl Acridinium NHS-ester (1 mg/ml in acetonitrile, InVent GmbH, Germany) (EP 0353971) and incubated for 20 min at room temperature. Labelled antibody was purified by gel-filtration HPLC on Bio-Sil SEC 400-5 (Bio-Rad Laboratories, Inc., USA) The purified labelled antibody was diluted in (300 mmol/l potassiumphosphate, 100 mmol/l NaCl, 10 mmol/l Na-EDTA, 5 g/l bovine serum albumin, pH 7.0). The final concentration was approx. 800.000 relative light units (RLU) of labelled compound (approx. 20 ng labeled antibody) per 200 µl. Acridiniumester chemiluminescence was measured by using an AutoLumat LB 953 (Berthold Technologies GmbH & Co. KG).

Solid phase antibody (coated antibody):
Solid phase: Polystyrene tubes (Greiner Bio-One International AG, Austria) were coated (18 h at room temperature) with antibody (1.5 µg antibody/0.3 ml 100 mmol/l NaCl, 50 mmol/l Tris/HCl, pH 7.8). After blocking with 5 % bovine serum albumine, the tubes were washed with PBS, pH 7.4 and vacuum dried.

Antibody specificity:
The crossreactivities of the different antibodies are listed in table 2.

**Table 2:**

| Peptide for immunization | Pre-Pro-Enkephalin-sequence | Antibody name |
|---|---|---|
| (C)DAEEDD | 119-125 (SEQ ID NO: 25) | NT-MRPENK |
| (C)EEDDSLANSSDLLK | 121-134 (SEQ ID NO: 26) | NM-MRPENK |
| (C)LKELLETG | 133-140 (SEQ ID NO: 27) | MR-MRPENK |
| (C)TGDNRERSHHQDGSDNE | 139-155 (SEQ ID NO: 28) | MC-MRPENK |
| (C)SDNEEEVS | 152-159 (SEQ ID NO: 29) | CT-MRPENK |

Antibody cross-reactivities were determined as follows: 1ug peptide in 300 µl PBS, pH 7,4 was pipetted into Polystyrene tubes and incubated for 1h at room temperature. After incubation the tubes were washed 5 times (each 1ml) using 5% BSA in PBS, pH 7,4. Each of the labelled antibodies were added (300 µl in PBS, pH 7.4, 800.000 RLU/ 300 µl) an incubated for 2h at room temperature, After washing 5 times (each 1ml of washing solution (20 mmol/l PBS, pH 7.4, 0.1 % Triton X 100), the remaining luminescence (labelled antibody) was quantified using the AutoLumat LB 953. MRPENK-peptide was used as reference substance (100%).

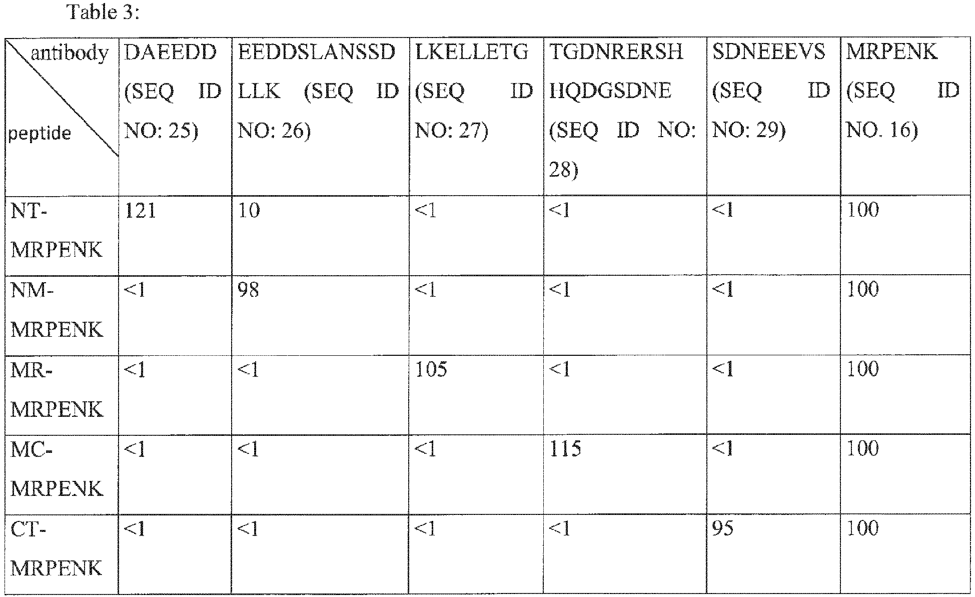

All antibodies bound the MRPENK peptide, comparable to the peptides which were used for immunization. Except for NT-MRPENK-antibody (10% cross reaction with EEDDSLANSSDLLK) no antibody showed a cross reaction with MR-PENK peptides not used for immunization of the antibody.

### Pro-Enkephalin Immunoassay:

50 µl of sample (or calibrator) was pipetted into coated tubes, after adding labeled antibody (200ul), the tubes were incubated for 2 h at 18-25 °C. Unbound tracer was removed by washing 5 times (each 1 ml) with washing solution (20 mmol/l PBS, pH 7.4, 0.1 % Triton X-100). Tube-bound labelled antibody was measured by using the Luminumeter LB 953 and a fixed concentration of 1000pmol/l of MRPENK. The signal (RLU at 1000pmol MRPENK/1) to noise (RLU without MRPENK) ratio of different antibody combinations is given in table 4. All antibodies were able to generate a sandwich complex with any other antibody. Surprisingly, the strongest signal to noise ratio (best sensitivity) was generated by combining the MR-MRPENK- and CT-MRPENK antibody. Subsequently, we used this antibody combination to perform the MRPENK-immunoassay for further investigations. MR-MRPENK antibody was used as coated tube antibody and CT-MRPENK antibody was used as labelled antibody.

**Table 4:**

| | Solid phase antibody | NT-MRPENK | NM-MRPENK | MR-MRPENK | MC-MRPENK | CT-MRPENK |
|---|---|---|---|---|---|---|
| Labelled antibody | | | | | | |
| NT-MRPENK | | / | 27 | 212 | 232 | <1 |
| NM-MRPENK | | 36 | / | 451 | 487 | <1 |
| MR-MRPENK | | 175 | 306 | / | 536 | 1050 |
| MC-MRPENK | | 329 | 577 | 542 | / | <1 |
| CT-MRPENK | | <1 | 615 | 1117 | 516 | / |

### Calibration:

The assay was calibrated, using dilutions of synthetic MRPENK, diluted in 20 mM K2PO4, 6 mM EDTA, 0,5% BSA, 50µM Amastatin, 100µM Leupeptin, pH 8.0. Pro-Enkephalin control plasma is available at ICI-diagnostics, Berlin, Germany. (Fig. 2)

The assay sensitivity was 20 determinations of 0-calibrator (no addition of MRPENK) + 2SD) 5,5 pmol/L.

### Example 4

### Malmö Diet and Cancer Study

The Malmö Diet and Cancer (MDC) study is a population-based, prospective epidemiologic cohort of 28449 men (born 1923-1945) and women (born 1923-1950) from the city of Malmö in southern Sweden who underwent baseline examinations between 1991 and 1996 (Minisymposium: The malmo diet and cancer study. Design, biological bank and biomarker programme. 23 october 1991, malmo, sweden. Journal of internal medicine 1993;233:39-79). From this cohort, 6103 men and women were randomly selected to participate in the MDC Cardiovascular Cohort (MDC-CC), which was designed to investigate the epidemiology of carotid artery disease, between 1991 and 1994 (Persson M, Berglund G, Nelson JJ, Hedblad B. Lp-pla2 activity and mass are associated with increased incidence of ischemic stroke: A population-based cohort study from malmo, sweden. Atherosclerosis 2008;200:191-8). Of women free from history and prevalence of breast cancer at the baseline exam fasted plasma samples for analysis of Pro-Neurotensin and Pro-Enkephalin and complete data on baseline covariates used in the fully adjusted model for incidence of breast cancer was available in 1929 women, as described in a recent previous study (Melander O, Maisel AS, Almgren P, Manjer J, Belting M, Hedblad B, et al. Plasma proneurotensin and incidence of diabetes, cardiovascular disease, breast cancer, and mortality. JAMA : the journal of the American Medical Association 2012;308:1469-75).

The baseline examination procedure has been described before (Belting M, Almgren P, Manjer J, Hedblad B, Struck J, Wang TJ, et al. Vasoactive peptides with angiogenesis-regulating activity predict cancer risk in males. Cancer epidemiology, biomarkers & prevention : a publication of the American Association for Cancer Research, cosponsored by the American Society of Preventive Oncology 2012;21:513-22 - Enhorning S, Wang TJ, Nilsson PM, Almgren P, Hedblad B, Berglund G, et al. Plasma copeptin and the risk of diabetes mellitus. Circulation 2010;121:2102-8 - Melander O, Newton-Cheh C, Almgren P, Hedblad B, Berglund G, Engstrom G, et al. Novel and conventional biomarkers for prediction of incident cardiovascular events in the community. JAMA : the journal of the American Medical Association 2009;302:49-57). Blood sampling was performed in the fasted state and -80°C EDTA plasma aliquots were stored. Body mass index (BMI) was defined as the weight in kilograms divided by the square of the height in meters. Data on smoking, educational level, current use of hormone replacement therapy as well as ever use of oral contraceptives (never/ever), age at menarche and number of children was ascertained from a questionnaire (Manjer J, Elmstahl S, Janzon L, Berglund G. Invitation to a population-based cohort study: Differences between subjects recruited using various strategies. Scandinavian journal of public health 2002;30:103-12). Educational level was divided into three categories: a maximum of 9 years of education, 9-12 years of education and completed university degree. Menopausal status was assessed with the help of the questionnaire and medical records as described previously (Butt S, Borgquist S, Anagnostaki L, Landberg G, Manjer J. Parity and age at first childbirth in relation to the risk of different breast cancer subgroups. International journal of cancer Journal international du cancer 2009;125:1926-34). Current smoking was defined as any cigarette smoking within the past year. During a median (interquartile range) follow-up time of 15.7 (15.1-16.2) years there were 123 incident cases of breast cancer among these 1929 women (Melander O, Maisel AS, Almgren P, Manjer J, Belting M, Hedblad B, et al. Plasma proneurotensin and incidence of diabetes, cardiovascular disease, breast cancer, and mortality. JAMA : the journal of the American Medical Association 2012;308:1469-75). The baseline characteristics of the study population are shown:

**Table 5:**

| | **Women in MDC (n=1929)** |
|---|---|
| **Age (years)** | 57.6 ± 5.9 |
| **Body Mass Index (kg/m²)** | 25.5 ± 4.2 |
| **Current smokers, n (%)** | 485 (25.1) |
| **Hormone replacement therapy, n (%)** | 463 (19.9) |
| **Ever use of oral contraceptives, n (%)** | 850 (44.1) |
| **Educational level, n (%)^{b}** | 1439 (74.6) / 141 (7.3) / 349 (18.1) |
| **Postmenopausal status, n (%)** | 1438 (74.5) |
| **Age at menarche (years)** | 13.6 ± 1.5 |
| **Number of children** | 1.8 ± 1.1 |
| **AHR, n (%)** | 322 (16.7) |

| | |
|---|---|
| *Data are given as means* ± *standard deviation. Categorical data are presented as numbers (percentages).* *^{b}Given as percentage of participants with a maximum of 9 years of education* / *9-12 years of education* / *completed university degree* | |

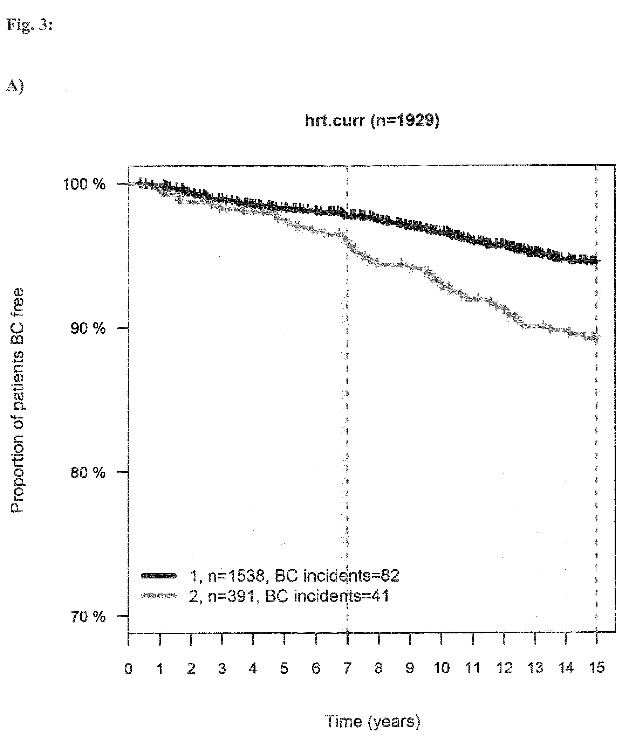

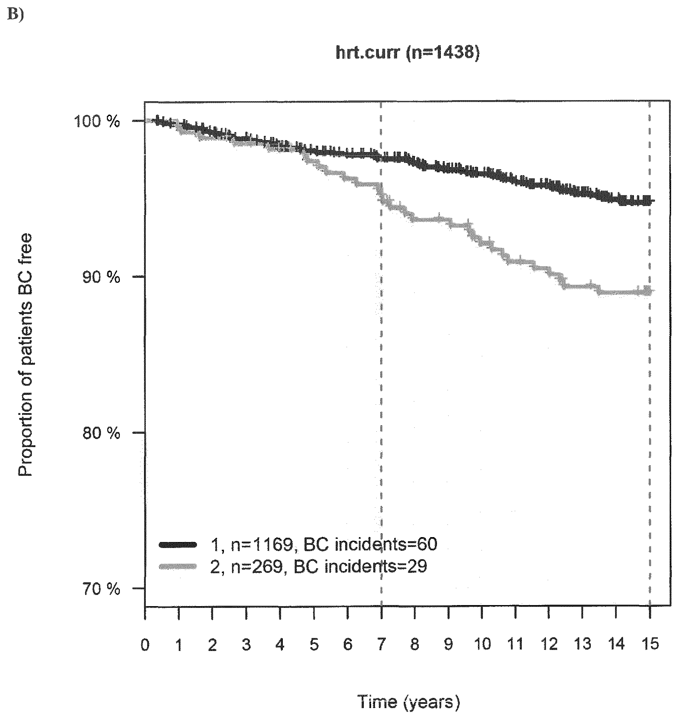

In line with previous results from others (Chlebowski RT, Hendrix SL, Langer RD, Stefanick ML, Gass M, Lane D, et al. Influence of estrogen plus progestin on breast cancer and mammography in healthy postmenopausal women: The women's health initiative randomized trial. JAMA : the journal of the American Medical Association 2003;289:3243-53. - Chlebowski RT, Manson JE, Anderson GL, Cauley JA, Aragaki AK, Stefanick ML, et al. Estrogen plus progestin and breast cancer incidence and mortality in the women's health initiative observational study. Journal of the National Cancer Institute 2013;105:526-35 - Beral V, Million Women Study C. Breast cancer and hormone-replacement therapy in the million women study. Lancet 2003;362:419-27), we found that women who received HRT at the time of inclusion (n=391) had a significantly higher risk to develop breast cancer than those who did not receive HRT (n=1538), both in univariate (HR[95%CI]=2.0 [1.38-2.92]; p=0.00051) and multivariate analysis (HR[95%CI]=2.01 [1.36-2.97]; p=0.0005, HR adjusted for age, use of antihypertensive medication, use of hormone therapy, ever use of oral contraceptives, educational level, age at menarche, number of children, menopausal status, systolic blood pressure, BMI, diabetes mellitus, current smoking, prevalent cardiovascular disease, heredity for cancer, and fasting concentrations of HDL, LDL and insulin (breast cancer risk factors). (Fig. 3 a and b)

At the time of inclusion, 491 women were pre- or perimenopausal, and 1438 were postmenopausal. Approximately one quarter in each group received HRT at inclusion (134 of 491 pre- or perimenopausal women (27.3 %), and 298 of 1438 postmenopausal women (20.7%)). The rate of incident breast cancer development was similar in both groups.

**Table 6:**

| | | **BC-** | **BC+** | **BC rate** |
|---|---|---|---|---|
| | | | | |
| **pre or peri** | **no HRT** | 369 | 22 | 5.6% |
| | **HRT** | 122 | 12 | 9.0% |
| | | | | |
| **post** | **no HRT** | 1169 | 60 | 4.9% |
| | **HRT** | 269 | 29 | 9.7% |

### pro-Neurotensin

Previously it has been shown that increasing fasting plasma Pro-Neurotensin (pro-NT) concentrations at inclusion are associated with an increased risk for the development of breast cancer (Melander O, Maisel AS, Almgren P, Manjer J, Belting M, Hedblad B, et al. Plasma proneurotensin and incidence of diabetes, cardiovascular disease, breast cancer, and mortality. JAMA : the journal of the American Medical Association 2012;308:1469-75, - Melander O, Belting M, Manjer J, Maisel AS, Hedblad B, Engstrom G, et al. Validation of plasma proneurotensin as a novel biomarker for the prediction of incident breast cancer. Cancer epidemiology, biomarkers & prevention : a publication of the American Association for Cancer Research, cosponsored by the American Society of Preventive Oncology 2014;23:1672-6). In all women, the Hazard risk for women with a pro-NT concentration above 190 pmol/L (=87^{th} percentile of population) compared to those below 190 pmol/L was 2.5 (CI 1.7-3.7, p<0.0001; AUC 0.57, CI 0.53-0.61). In postmenopausal women who received HRT, the Hazard risk for women with a pro-NT concentration above 190 pmol/L compared to those below 190 pmol/L was 4.1 (CI 1.9-8.7, p<0.0001; AUC 0.63, CI 0.54-0.71).

Similar results for other cut points (all p<0.001):
- Cut point 130 pmol/L (65^{th} percentile): HR(all)=1.9, HR(postmenopausal plus HRT)=2.2
- Cut point 160 pmol/L (79^{th} percentile): HR(all)=2.0, HR(postmenopausal plus HRT)=3.0

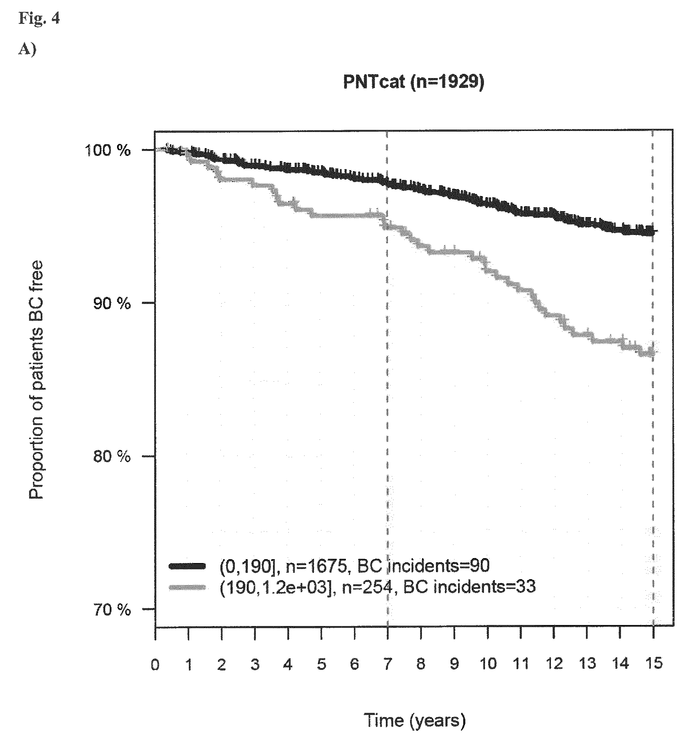

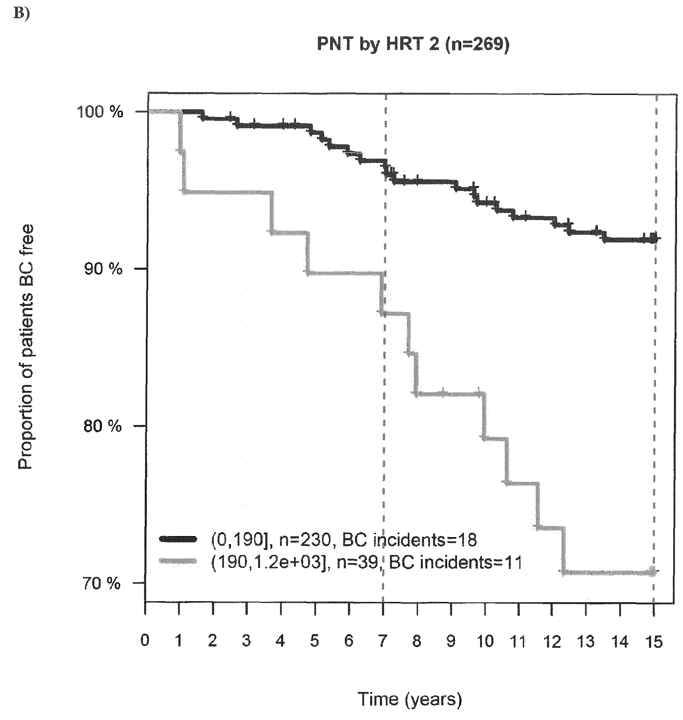

These results demonstrate that risk stratification by plasma pro-Neurotensin is approximately twice as strong in postmenopausal women receiving HRT compared to all women. (Fig. 4 a and b)
Median concentrations of pro-NT did not differ between all women and those who received HRT.

### Pro-Enkephalin

Previously it has been shown that decreasing fasting plasma Pro-Enkephalin (pENK) concentrations at inclusion are associated with an increased risk for the development of breast cancer (WO2014/053502; WO2013/132089). In all women, the Hazard risk for women with a pro-ENK concentration above 46 pmol/L (=45.5^{th} percentile) compared to those below 46 pmol/L was 0.47 (CI 0.33-0.68, p<0.0001; AUC 0.59, CI 0.55-0.63). In postmenopausal women who received HRT, the Hazard risk for women with a pro-ENK concentration above 46 pmol/L compared to those below 46 pmol/L was 0.23 (CI 0.09-0.57, p=0.0003; AUC 0.66, CI 0.58-0.74).

Similar results for other cut points (all p<0.001):
- Cut point 44 pmol/L (37.6^{th} percentile): HR(all)=0.60, HR(postmenopausal plus HRT)=0.33
- Cut point 48 pmol/L (53.2^{nd} percentile): HR(all)=0.47, HR(postmenopausal plus HRT)=0.31

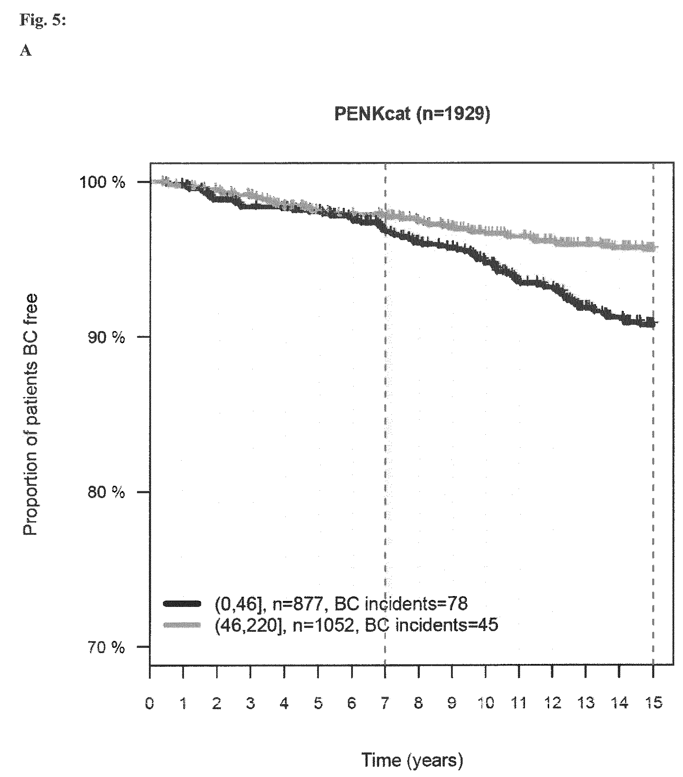

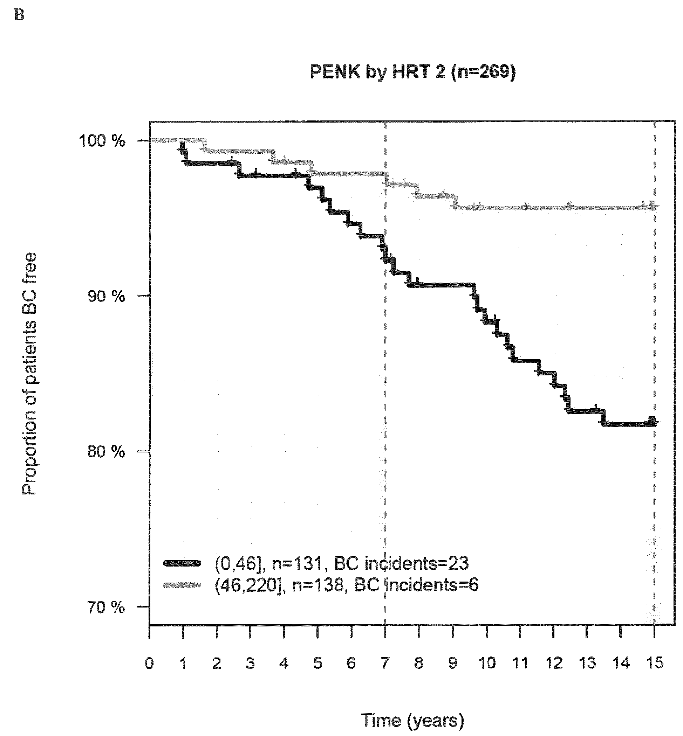

These results demonstrate that risk stratification by plasma Pro-Enkephalin is approximately twice as strong in postmenopausal women receiving HRT compared to all women. (Fig. 5 a and b)
Median concentrations of pENK did not differ between all women and those who received HRT.

### Combination of Pro-Neurotensin and Pro-Enkephalin

We assessed whether the combination of Pro-Neurotensin and Pro-Enkephalin further improves risk prediction for incident breast cancer in comparison to one of the markers only.

As an example for a possible combination, three risk groups were defined as follows:
First, "high-" and "low-risk"-groups were defined for the two markers separately:
   A Pro-Neurotensin "high risk"-group was defined as subjects with Pro-Neurotensin concentrations above 190 pmol/L, and a Pro-Neurotensin "low risk"-group was defined as subjects with Pro-Neurotensin concentrations below 190 pmol/L.

A Pro-Enkepahlin "high risk"-group was defined as subjects with Pro-Enkepahlin concentrations below 46 pmol/L, and a Pro-Enkepahlin "low risk"-group was defined as subjects with Pro- Enkepahlin concentrations above 46 pmol/L.

For the combination of both markers, a "low risk"-group was defined as subjects being in the "low risk"-group of both markers, a "medium risk"-group was defined as subjects being in the "low risk"-group of one marker and in the "high risk"-group of the other marker, and a "high risk"-group was defined as subjects being in the "high risk"-group of both markers.

In all women, the Hazard risk of women in the high-risk group vs. women in the low risk group was 5.6 (CI 3.3-9.6, p<0.0001; AUC 0.63). In postmenopausal women who received HRT, the Hazard risk of women in the high-risk group vs. women in the low risk group was 18.2 (CI 5.6-59.3, p<0.0001; AUC 0.72).

These results demonstrate that risk stratification by combining plasma Pro-Neurotensin and plasma Pro-Enkephalin is three times as strong in postmenopausal women receiving HRT compared to all women. (Fig. 6 a and b)

### Figure description

Fig. 1: Calibration curve PNT. Figure 1 shows a typical P-NT dose/ signal curve.
Figure 2 shows a typical Pro-Enkephalin dose / signal curve. Standard curve Pro-Enkephalin.
Figure 3 a and b: Kaplan Meier Plot of incident breast cancer diagnoses depending on whether (2) or not (1) women received HRT at inclusion for all women (A) and the subgroup of postmenopausal (B).
Figure 4 a and b: Kaplan Meier Plot of incident breast cancer diagnoses depending on plasma pro-Neurotensin at a cut point of 190 pmol/L for all women (A) and in the subgroup of postmenopausal women receiving HRT (B). Below 190 pmol/L = black curves; above 190 pmol/L = grey curves.
Figure 5 a and b: Kaplan Meier Plot of incident breast cancer diagnoses depending on plasma Pro-Enkephalin at a cut point of 46 pmol/L for all women (A) and the subgroup of postmenopausal women receiving HRT (B). Below 46 pmol/L = black curves; above 46 pmol/L = grey curves.
Figure 6 a and b: Kaplan Meier Plot of incident breast cancer diagnoses depending on three risk groups defined by certain combinations of plasma Pro-Neurotensin and Pro-Enkephalin as described for all women (A) and the subgroup of women with HRT (B).

## Claims

1. A method for stratifying a female subject for hormone replacement therapy comprising:
• determining the level of Pro-Neurotensin or fragments thereof of at least 5 amino acids in a bodily fluid obtained from said female subject; and
• comparing said level of Pro-Neurotensin or fragments thereof with a pre-determined "pro-NT threshold" and
• wherein in case the determined level of Pro-Neurotensin or fragments thereof is above said pre-determined threshold then the subject is identified as having an enhanced risk to suffer an adverse event or having an enhanced risk to attract a disease in case of a hormone replacement therapy, and
• wherein in case the determined level of Pro-Neurotensin or fragments thereof is below said pre-determined threshold then the subject is identified as not having an enhanced risk to suffer an adverse event or as not having an enhanced risk to attract a disease in case of a hormone replacement therapy, and/or
• determining the level of Pro-Enkephalin or fragments thereof of at least 5 amino acids (pENK) in a bodily fluid obtained from said female subject; and
• comparing said level of Pro-Enkephalin or fragments thereof with a pre-determined "pENK threshold" and
• wherein in case the determined level of Pro-Enkephalin or fragments thereof is below said pre-determined pENK threshold then the subject is identified as having an enhanced risk to suffer an adverse event or having an enhanced risk to attract a disease in case of a hormone replacement therapy, and
• wherein in case the determined level of Pro-Enkephalin or fragments thereof is above said pre-determined pENK threshold then the subject is identified as not having an enhanced risk to suffer an adverse event or as not having an enhanced risk to attract a disease in case of a hormone replacement therapy .

2. A method for stratifying a female subject for hormone replacement therapy according to claim 1 wherein said adverse event or disease is selected from the group comprising, diabetes, metabolic syndrome, cardiac event, cardiac disease, and cancer in particular breast cancer or lung cancer.

3. A method for stratifying a female subject for hormone replacement therapy according to claim 1 or 2 wherein said female subject is either going through menopause or is post-menopausal.

4. A method for stratifying a female subject for hormone replacement therapy according to any of claims 1 to 3, wherein said subject does not suffer from cancer, in particular breast cancer or lung cancer.

5. A method for stratifying a female subject for hormone replacement therapy according to any of claims 1 to 4, wherein said subject does not suffer from diabetes.

6. A method for stratifying a female subject for hormone replacement therapy according to any of claims 1 to 5, wherein said subject does not suffer from metabolic syndrome.

7. A method for stratifying a female subject for hormone replacement therapy according to any of claims 1 to 6, wherein said subject does not have suffered a cardiac event.

8. A method for stratifying a female subject for hormone replacement therapy according to any of claims 1 to 7, wherein said subject does not have suffered a cardiac disease.

9. A method for stratifying a female subject for hormone replacement therapy according to any of claims 1-8 wherein said female subject has not yet received hormone replacement therapy.

10. A method for stratifying a female subject for hormone replacement therapy wherein said subject does not suffer from cancer according to any of claims claim 1 or 9 wherein said female subject is receiving hormone placement therapy.

11. A method for stratifying a female subject for hormone replacement therapy wherein said subject does not suffer from cancer according to any of claims claim 1 to 10 wherein in case the level of Pro-Neurotensin or fragments thereof is higher than said pre-determined pro-NT threshold and / or if the level of Pro-Enkephalin or fragments thereof of at least 5 amino acids is lower than said pre-determined pENK threshold then hormone replacement therapy is either withhold from said female subject or hormone replacement therapy will be stopped in said female subject.

12. A method for stratifying a female subject for hormone replacement therapy wherein said subject does not suffer from cancer according to any of claims claim 1 to 11 wherein Pro-Neurotensin or fragments thereof is selected from Pro-Neurotensin 1-117, fragments of Pro-Neurotensin 1-117 (SEQ ID NO: 5) of at least 5 amino acids or Pro-Neurotensin 1-117 (SEQ ID NO: 5) comprising peptides.

13. A method for stratifying a female subject for hormone replacement therapy wherein said subject does not suffer from cancer according to any of claims claim 1 to 12 wherein Pro-Enkephalin or fragments thereof is selected from the group comprising MRPENK (MRPENK is SEQ ID NO. 16: Pro-Enkephalin 119-159, Mid regional Pro-Enkephalin-fragment, MRPENK). Fragments of MRPENK of at least 5 amino acids or MRPENK comprising peptides.

14. A method according to any of claims 1 to 13, wherein the level of Pro-Neurotensin or fragments thereof of at least 5 amino acids and / or the level of Pro-Enkephalin or fragments thereof of at least 5 amino acids comprising peptides in a bodily fluid is the fasting level.

15. A method according to any of claims 1 to 13, wherein said female subject has never had a history of diagnosis of cancer at the time the sample of bodily fluid is taken from said female subject.

16. A method according to any of claims 1 to 15, wherein additionally at least one parameter is determined selected from the group comprising: age, presence of diabetes mellitus, current smoking, Time since onset of menopause, LDL cholesterol, LDL/HDL cholesterol ratio, presence of metabolic syndrome, and presence of factor V Leiden genotype, body mass index, breast density, race/ethnicity.

17. A method according to any of the preceding claims, wherein the level of MR-Pro-Enkephalin (SEQ ID No. 16) and/or Pro-Neurotensin 1-117 (SEQ ID NO: 5) is determined.

18. A method according to any of the preceding claims, wherein the level of Pro-Neurotensin or fragments thereof and / or the level of Pro-Enkephalin or fragments thereof of at least 5 amino acids is measured with an immunoassay.
